# EUROPEAN PATENT APPLICATION

(11) **EP 4 850 593 A1**
(43) Date of publication of application: **09.12.2026**
(21) Application number: 25180929.9
(22) Date of filing: 05.06.2025
(51) Int. Cl.: A61B 5/00

(54) **A PRESSURE SENSOR ASSEMBLY FOR MEASURING THE PRESSURE OF A FLUID IN A BODILY DUCT OF A PATIENT AND A CATHETER HAVING SUCH A PRESSURE SENSOR ASSEMBLY**

(71) Applicant: PulmoTech B.V., 9351 VK Leek (NL)
(72) Inventor: FLINK, Rutger Christiaan, 5263 EX Vught (NL)
(74) Representative: Hepp Wenger Ryffel AG

(57) **Abstract**

A pressure sensor assembly (10) for measuring the pressure of a fluid in a bodily duct of a patient is used for measuring the gas pressure in a measuring region (R). The sensor assembly (10) comprises a sensor mount (12) and a pressure sensor (14) mounted on the sensor mount. At least one end region (15) of the pressure sensor is not supported by the sensor mount (12).

## Description

The invention relates to a pressure sensor assembly for measuring the pressure of a fluid in a bodily duct of a patient and a catheter with such a pressure sensor assembly according to the preamble of the independent patent claims.

Pressure measurement catheters for measuring the pressure in a bodily duct such as the esophagus are known in the art.

According to WO 2020/173948, such a pressure measurement catheter is e.g. provided with a pressure sensor in the form of a pressure transducer. The pressure transducer is arranged inside the lumen of the catheter.

In known catheters, pressure sensors are mounted on a sensor mount, e.g. formed by a printed circuit board. Due to a rigid connection between the pressure sensor, the sensor mount and an external housing, external forces acting on the sensor mount or on the housing may cause a stress on the pressure sensor.

Polymers used for making catheters or adhesives for mounting the pressure transducer within the catheter may lead to moisture absorption during clinical use due to their hygroscopic properties. During use, a swelling hence can occur. Also, thermal stresses might occur due to the difference of temperature inside and outside of the patient's body. Swelling, thermal stresses or external forces acting onto the catheter may cause internal mechanical stresses onto a sensor mounted within the catheter.

Such stresses can induce strain on the sensing element of the pressure sensor and hence can affect the accuracy of pressure measurements and can be the source of drift over time.

It is an object of the present invention to overcome the drawbacks of the prior art and in particular to provide a pressure sensor assembly and a catheter having an improved accuracy of pressure measurements and in particular with a reduced stress induced signal drift due to moisture absorption when inserted into a bodily cavity such as the trachea or the esophagus.

According to the present invention, these objects are solved with a pressure sensor assembly and a catheter according to the characterising portion of the independent claims.

According to the invention, a pressure sensor assembly for measuring the pressure of a fluid in a bodily duct of a patient is provided. The pressure sensor assembly is in particular suitable for measurement of a gas pressure and typically can be used for an endotracheal, an esophageal or a gastric tube or combinations thereof. The sensor assembly comprises a sensor mount. At least one pressure sensor is mounted on the sensor mount. More than one pressure sensor could be mounted on the sensor mount. Also, more than one sensor assembly could be mounted in one catheter.

According to the invention, at least one end region of the pressure sensor is not supported by said sensor mount. This end region is hence free to move or to deflect under environmental or material-induced stresses. Such a floating configuration of the pressure sensor reduces mechanical effects on the pressure sensor due to e.g. moisture-absorption induced stresses or due to external forces acting on other components of the pressure sensor assembly. The pressure sensor is hence mechanically decoupled at least partially from the sensor mount and/or from a housing.

The sensor mount may be any structure onto which the pressure sensor is mounted. Preferably, the sensor mount is formed by a printed circuit board.

The printed circuit board can be itself flexible and the pressure sensor can be directly mounted in a one-sided manner onto the printed circuit board. The mounting can be made with a relatively soft die attach adhesive. Preferably, the die attach adhesive has a Shore D hardness of 75 to 90. Typically, a printed circuit board is considered flexible if it is made of one or two layers of e.g. Polyimide, able to provide bending radii of below 2 mm without breaking or damaging the copper structures that form the wiring.

Alternatively, the printed circuit board can be rigid, and the pressure sensor is directly mounted in a one-sided manner with a soft die attach adhesive which preferably has a Shore D hardness of 50 to 80. Typically, a printed circuit board is considered rigid is e.g. made of a rigid FR4 glass-reinforced epoxy laminate material, not intended to be bendable to radii below 2 centimetres without breaking.

The printed circuit board primarily serves the mounting of the pressure sensor. It can further provide additional or alternative functions. It may provide a wire connection for a signal from the pressure sensor to a connection point of the catheter for transmitting signals outside of the patient's body.

It may also provide attachment points for attaching a separate wire which transmits the signal to a connection point of the catheter.

Connecting wires can be attached to such attachment points by soldering, gluing or crimping.

When the pressure sensor is mounted on a printed circuit board, the pressure sensor can be connected to the printed circuit board with bond wires. The bond wires can be encased in a flexible glob top material. For this purpose, preferably a glob top material having a Shore D hardness of below 80 can be used. More preferably, a material having a Shore D hardness below 65 and most preferably below 55 can be used. The flexible glob top material for covering the bond wires may be e.g. an epoxy, a silicone, or an acrylic material, such as acrylic urethane.

Alternatively to the direct mounting described above, it is also possible to mount the pressure sensor indirectly on a sensor mount, typically exclusively via the bond wires. In this case, the bond wires together with a very soft glob top encasing provide the decoupled mounting alone.

The pressure sensor can have a proximal end and a distal end. According to a preferred embodiment, the pressure sensor is mounted on the sensor mount exclusively at its proximal end. It is hence arranged unsupported at its distal end. This allows a free-floating arrangement of an active measuring surface of the pressure sensor which is arranged between its proximal and its distal end. The terms proximal / distal here refer to the areas of the sensor which are closer / further away from an operator or from a measuring unit to which the pressure sensor assembly is attached.

Preferably, the pressure sensor is exclusively supported on one side of the measuring surface, preferably its proximal side, whereas the other side of the measuring surface remains unsupported.

Typically, depending on the size of the sensor, the unsupported end region of the pressure sensor has a length of at least 0.6 mm. Alternatively or additionally, the pressure sensor is typically unsupported over at least 50% of its entire length, preferably over 75% of its length.

The pressure sensor assembly preferably is further provided with a sensor housing which at least partially encases the pressure sensor. Such a housing mechanically protects the pressure sensor and further allows an easy mounting of the pressure sensor assembly into another structure such as a catheter.

The sensor housing can be made of any suitable material. The sensor housing should be sufficiently rigid so that external stresses can not directly influence the pressure sensor and it should be sufficiently tight so that humidity and/or pressure differences may be kept away from the pressure sensor.

Typically, biocompatible materials are used for the sensor housing, such as steel, titanium or alloys thereof.

The sensor housing can be made of casted metal (e.g. by metal injection molding) or by stamped, molded metal, or machined metal or by combination thereof.

It is also possible to use stiff and strong plastics such as PEEK which can be molded or machined.

Furthermore, it is possible to use ceramics which can be molded or casted from slurries or powder and be sintered, if needed.

The sensor housing can be provided with an opening which can be directed towards a measuring region where the pressure, preferably a pressure of a gas, shall be measured. The measuring surface of the pressure sensor is arranged within this opening so that the measuring surface can be impinged by the pressure in the measuring region.

Preferably, the pressure sensor is at least partially encased by the sensor housing, preferably in a sealing manner. Such a sealing encasing preferably can be achieved with a flexible adhesive material which is arranged between free edges of the housing and the pressure sensor. Preferably, a periphery of the measuring surface of the pressure sensor is sealingly mounted within the opening of the housing.

According to a further aspect of the invention, a pressure sensor assembly for measuring the pressure of a fluid in a bodily duct of a patient is provided. The sensor assembly comprises a sensor mount and a pressure sensor mounted on the sensor mount. It further comprises a sensor housing which at least partially encases the pressure sensor.

The sensor housing is arranged in such a way that at least one end region of the sensor housing is not supported.

Preferably the sensor housing is connected to the sensor mount only at one end region, preferably with an adhesive. The area where the sensor housing is attached may be also distant from one end of the sensor housing. Depending on the chosen materials, the connections could also be made by welding operations, such as ultrasound welding, especially if the sensor housing is made from a plastic material.

The sensor housing is hence preferably mounted on the sensor mount by gluing at one fixation point only. The pressure sensor then can be mounted into the sensor housing by using soft adhesives for encapsulation to avoid stress due to differences in thermal expansion coefficients and/or swelling of adhesives.

While it is understood that such an arrangement of a housing is particularly preferable in context with a free-floating arrangement of a pressure sensor on the sensor mount as described herein above, such a partially supported arrangement of the housing may also be advantageous in context with pressure sensors which are rigidly mounted.

If an end of the housing remains unsupported and is free to move or deflect slightly under environmental stresses (e.g**.** a mechanical contact) or under material induced stresses (e.g**.** due to moisture), the accuracy can be further improved. In other words, according to this aspect of the invention, a spring-board-like mechanical decoupling is achieved. The sensor housing is anchored at only one end and is allowed to flex at its other end.

As a result of a one-sided mounting of the pressure sensor and/or of the sensor housing, swelling or deformation of the surrounding materials (which may be materials of the sensor assembly as such or materials in the vicinity of the sensor assembly) translate only minimally into a mechanical pressure acting on the pressure sensor. Signal drift is hence significantly reduced, and the stability is improved.

The housing as such is not rigidly mechanically fixed to the sensor mount, e.g. a printed circuit board. Preferably, a support for the housing such as a printed circuit board terminates before or beneath the unsupported end of the sensor housing.

Preferably, depending on the size, the sensor housing is only supported or connected to the sensor mount in the 30 - 40 proximalmost percent of its length. In other words, the sensor housing is unsupported on the sensor mount over the 60 to 70 distalmost percent, preferably over about the 75 distalmost percent. Alternatively or additionally, again depending on the size, the sensor housing is only supported along a length necessary for a sufficient amount of glue for fixing the sensor housing, typically with a diameter of 1 to 1.5 mm in the longitudinal direction.

The pressure sensor can be encapsulated in the housing with a soft adhesive such as silicone.

The sealing encapsulation preferably is liquid tight and airtight, so that pressure difference measurements can be made with an appropriate pressure sensor.

Preferably, the pressure sensor is formed by a MEMS structure. Such MEMS based pressure sensors (Micro-Electro-Mechanical-Systems) can be made by using semi-conductor microfabrication techniques similar to those used for manufacturing integrated circuits. In addition, mechanical structures such as membranes or beams can be added making such MEMS suitable for differential pressure measurements.

Typically, a MEMS pressure sensor consists of a substrate which forms the foundation layer where all other structures are built on. The substrate is typically silicon. A thin, flexible membrane forming a diaphragm is edged into or deposited onto the substrate. The diaphragm deflects under pressure.

A sensing element is used to detect diaphragm movement. Different types of sensing elements are known and can be used in the context of the present invention.

Piezo-resistive sensing elements include resistors embedded in the diaphragm which change their resistance when the membrane flexis.

Capacity sensing elements have a diaphragm which forms one plate of a capacitor. The deflection of the diaphragm changes the capacitance.

In piezo electric sensing elements, the diaphragm is coated with a piezo electric material, which generates a voltage when put under stress.

In cavity sensing elements, a sealed cavity underneath the diaphragm acts as a reference vacuum or gas chamber.

It is also possible to use open-to-ambient sensors where one side of the diaphragm is open to atmospheric pressure via a hole in the substrate or in the chip package.

Alternatively, it is also possible to use optical sensors such as fibre optic sensors (e.g. Fabri Pérot interferometers with a thin diaphragm to reflect light and thus having a cavity length which changes with pressure) or fibre Bragg gratings deforming under pressure.

According to a preferred embodiment, a full Wheatstone bridge sensor can be used. Resistors are arranged in the form of a Wheatstone bridge in a piezoresistive sensing element. A voltage signal proportional to the existing pressure can thereby be generated and the signal can be amplified. In a full bridge configuration, temperature drift can be compensated.

According to still another aspect of the invention, a catheter for insertion into a bodily cavity of a patient and for measuring the pressure in a measuring region of the bodily cavity is provided. The catheter has a wall which defines at least one inner lumen. The catheter further comprises at least one pressure sensor assembly as previously described. The pressure sensor assembly is arranged such that it can be impinged by the pressure in the pressure measuring region. It is also possible to provide more than one pressure sensor assembly as disclosed e.g. in WO 2020/173948 and/or to provide the catheter with spacing elements in the form of balloons or other structures such as described in WO 2020/173948.

Preferably, the catheter is provided with at least one window. The pressure sensor assembly is arranged within the lumen of the catheter adjacent to this window such that a measuring surface of the pressure sensor is impinged by the pressure in the measuring region.

Preferably, the catheter is provided with electrical conductors arranged within the lumen for connecting the pressure sensor assembly to a measuring unit which is arranged outside of the patient's body, similarly to the one described in WO 2020/173948.

With a pressure sensor assembly and a catheter as described above, inaccuracies or signal drifts due to humidity can be reduced. For further improving accuracy, the catheter according to the invention can be additionally conditioned by subjecting it to an environment with a well-defined humidity for a certain period of time prior to insertion into the patient's body.

The invention will now be explained with reference to non-limiting embodiments and the drawings, which show:
- Figure 1:: A schematic representation of a pressure sensor assembly according to the prior art,
- Figure 2:: The pressure sensor assembly of figure 1, provided with an additional sensor housing,
- Figure 3:: A schematic representation of a pressure sensor assembly according to a first embodiment of the invention,
- Figure 4:: A schematic representation of a pressure sensor assembly according to a second embodiment of the invention,
- Figure 5:: A schematic representation of the embodiment of figure 4 with an additional sensor housing,
- Figure 6:: A schematic, perspective representation of a part of the pressure sensor assembly according to figure 4,
- Figure 7a:: An enlarged schematic view of a part of the pressure sensor assembly according to figure 5,
- Figure 7b:: A schematic cross-sectional view of the embodiment of figure 5 and 7a,
- Figure 8:: A schematic representation of a catheter according to the invention and
- Figure 9: The drift of a sensor assembly according to the prior art as shown in Figure 2 as compared to the drift of an embodiment according to Figure 5 and 6.

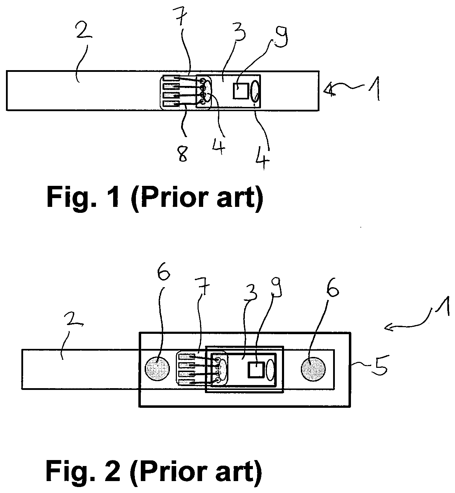

Figure 1 schematically shows a pressure sensor assembly 1 according to the prior art. The pressure sensor assembly 1 has a substrate 2 onto which a pressure sensor 3 is mounted. The substrate 2 is formed by a printed circuit board. Wire bonds 8 connect contacts of the printed circuit board 2 to the pressure sensor 3. The wire bonds 8 are encased with a glob top material 7. The pressure sensor 3 is rigidly mounted onto the substrate 2 at its both ends in two e.g. ellipsoid die attach areas by means of glue 4. A pressure measurement surface 9 is arranged between the two ends of the pressure sensor 3 where the pressure sensor 3 is glued onto the substrate 2.

Figure 2 shows the embodiment of figure 1 mounted in a sensor housing 5. The sensor housing 5 is attached to the substrate 2 by means of two points of glue 6. Due to the rigid mounting of the pressure sensor 3 on the substrate 2 and due to the rigid attachment between the sensor housing 5 and the substrate 2, external stresses acting on the sensor housing 5 and/or on the substrate 2 and/or internal stresses occurring within the glue 4/6 e.g. due to changes in moisture content cause strain onto the pressure sensor 3 and in particular its measurement surface 9. Measurement inaccuracies and drift result thereby.

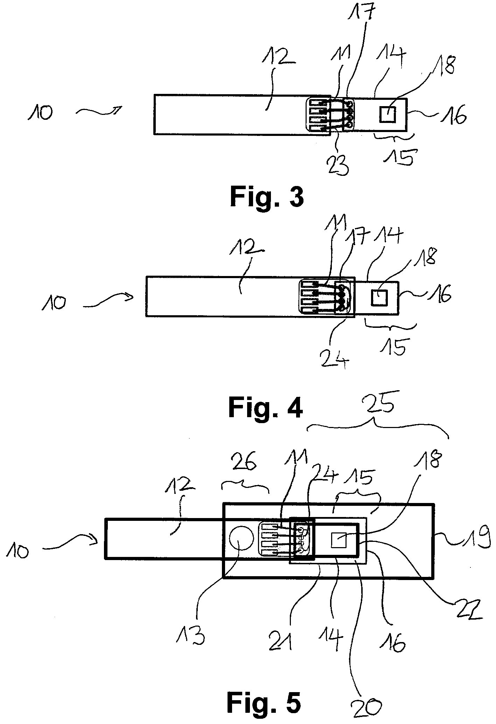

Figure 3 shows a first embodiment of a pressure sensor assembly 10 according to the invention. A pressure sensor 14 is mounted on a sensor mount 12 formed by a printed circuit board. The pressure sensor 14 has a proximal end 17 and a distal end 16. In an end region 15 neighbouring the distal end 16, the pressure sensor 14 is unsupported and free floating.

The pressure sensor 14 is only attached to the sensor mount 12 in a region neighbouring its proximal end 17. The pressure sensor 14 is connected to the printed circuit board 12 by means of bond wires 11. The bond wires are encased in a glob top material 23. The bond wires 11 both provide an electrical and a mechanical connection between the printed circuit board 12 and the pressure sensor 14. Due to the flexibility of the glob top material 23 and of the bond wires 11, the pressure sensor 14 and in particular a measuring surface 18 thereof is decoupled from the printed circuit board 12.

The printed circuit board 12 is made as a rigid board. The glob top material used is e.g. epoxy, silicone, acrylic (structural, e.g. acrylic urethan), having a Shore D hardness of preferably below 80, more preferable below 65 and most preferably below 55, typically an acrylic urethane with a Shore D hardness of 51.

Figure 4 shows an alternative embodiment to the embodiment of figure 3. The same reference numbers designate the same elements. The embodiment of figure 4 is different from the one of figure 3 in that the pressure sensor 14 is mounted onto the printed circuit board 12 in one e.g. ellipsoid die attach area with an adhesive material 24, e.g. an epoxy with a preferred Shore D hardness of 50 to 90. In the shown embodiment, the adhesive material 24 is an epoxy-based adhesive having a Shore D hardness of about 76 to 90 (depending on the curing) glued onto a flexible printed circuit board. The attachment is made in one end region of the pressure sensor 14 neighbouring its proximal end 17.

A measuring surface 18 is arranged in an end region 15 of the pressure sensor 14 neighbouring the distal end 16. The end region 15 is unsupported and free floating in the same manner as described with reference to figure 3.

In the embodiment of figure 4, the printed circuit board is in the form of a flexible board.

Figure 5 shows the embodiment of figure 4 which is provided with a sensor housing 19 which sealingly encases the pressure sensor 14. The sensor housing 19 is attached to the printed circuit board 12 with an adhesive material 13 only at a proximal end region 26. A distal end region 25 of the sensor housing 19 remains free floating with respect to the printed circuit board 12.

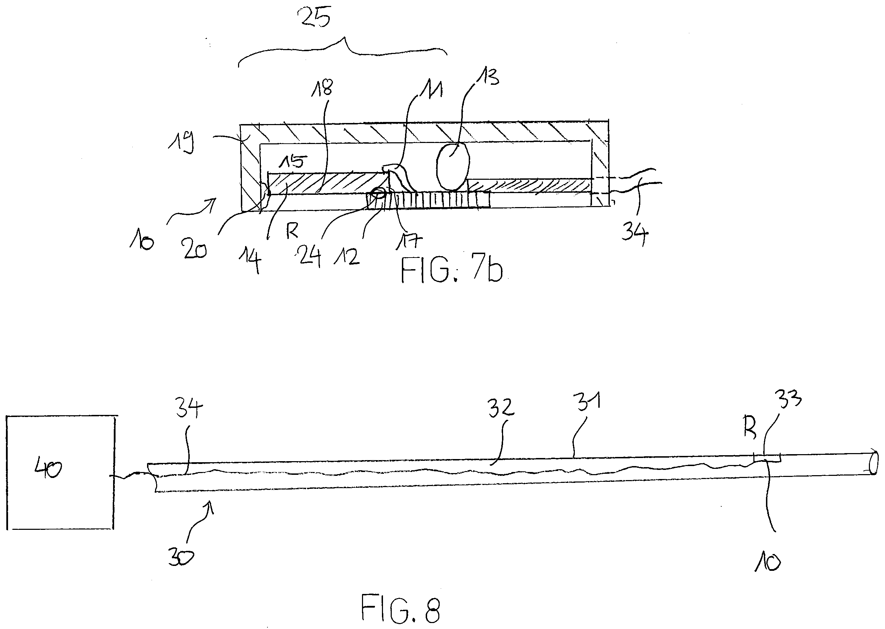

The sensor housing 19 is provided with an opening 21 which allows the pressure measuring surface 18 of the pressure sensor 14 to be impinged by external pressure of a measuring region R (see figures 7b and 8).

A gap between the boundaries of the opening 21 and of a periphery 22 around the measurement surface 18 of the pressure sensor 14 is sealed by means of an adhesive 20, e.g. with a soft biocompatible silicone, typically with a Shore A hardness of 15 - 20. The adhesive 20 seals off the front part of the pressure sensor 14 from the back to provide a balanced pressure measurement (balanced to ambient pressure, because the back of the sensor is open via the catheter lumen 32 to ambient, see figure 8).

The flexible adhesive preferably completely encases the sensor element 14 within the housing 19. Due to the flexibility of the adhesives 20, the pressure sensor 14 remains mechanically decoupled from the housing 19 despite the tight sealing.

Due to (i) the only one sided mounting of the pressure sensor 14 and of the housing 19, (ii) the flexible adhesives 24, 20 mounting the pressure sensor 14 on the sensor mount 12 and in the housing 19 and (iii) to the flexible adhesive 13 mounting the sensor housing 19 onto the printed circuit board 12, the pressure sensor 14 is not affected by any stresses onto the printed circuit board 12 and/or the sensor housing 19.

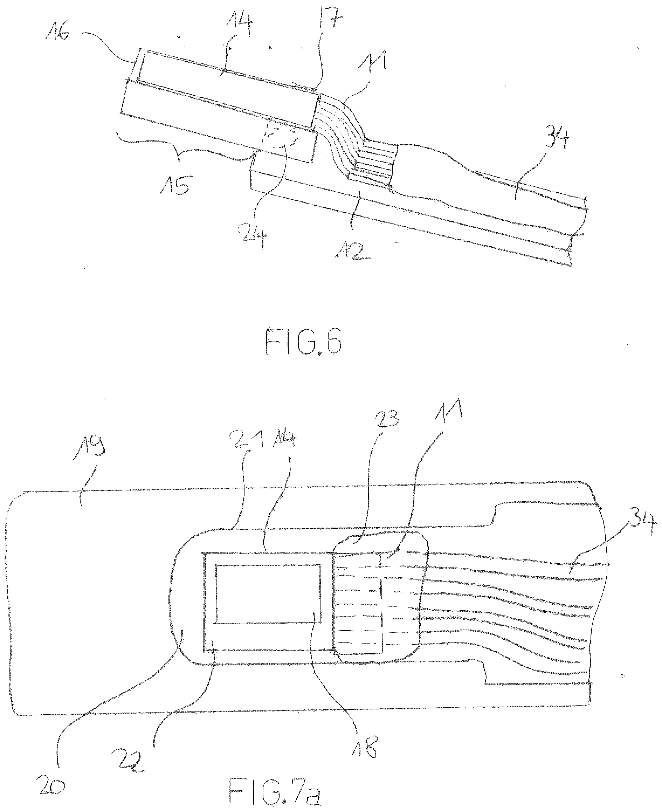

Figure 6 shows a schematic, perspective representation of the embodiment shown in figure 4. As it can be seen in figure 6, the end region 15 of the pressure sensor 14 adjacent to its distal end 16 is free floating and not supported by the printed circuit board 12.

The pressure sensor 14 is formed by a MEMS with a piezoresistive sensing element having a full Wheatstone bridge configuration.

The pressure sensor 14 is mounted to the printed surface board 12 at its proximal end 17 by means of the adhesive 24. The bond wires 11 electrically connect the pressure sensor 14 to the printed circuit board 12. An electrical conductor 34 is also attached to attachment points of the printed circuit board 12 contacting the bond wires 11 for transmitting signals provided by the pressure sensor 14 to an external measuring unit 40 (see figure 8).

Figure 7a shows an enlarged view on the measuring surface 18 of a pressure sensor 14 mounted in a housing 19 as shown in figure 5. The gap between the periphery 22 around the measuring surface 18 and the boundary of the opening 21 is filled by the adhesive 20. A glob top 23 is provided over the bond wires 11. The glob top material 23 and the sealing adhesive material 20 are individually chosen: The glop top 23 has a higher hardness than the sealing adhesive material 20.

Figure 7b shows a schematic cross-sectional view through the embodiment of figure 7a. As can be seen in figure 7b, the pressure sensor 14 is free floating at its distal end region 15 and is only attached to the printed circuit board 12 by means of an adhesive 24 at its proximal end 17. The sensor housing 19 is only attached to the printed circuit board 12 by means of one point of adhesive 13. The measuring surface 18 is open to the ambient pressure and can hence determine the pressure in a measuring region R. The gap between the housing 19 and the pressure sensor 14 is sealed by means of the adhesive 20.

Figure 8 schematically shows a catheter 30 according to the invention. The catheter 30 has a wall 31 defining a lumen 32. A window 33 is provided in the wall 31. A pressure sensor assembly 10 is arranged within the catheter 30 and sealing attached within the window 33 so that the pressure in a neighbouring measuring region R can be measured by the pressure sensor assembly 10. A connecting wire 34 connects the pressure sensor assembly 10 to an external measuring unit 40.

The lumen 32 is sealed off at the distal end of the catheter so that balancing against ambient pressure can be achieved.

Several pressure sensor assemblies 10 could be arranged along the length of the lumen 32, as shown in WO 2020/173948. In such a case, the printed circuit board 12 or the conductors 34 PCB might continue underneath a first sensor assembly 10 to reach a second sensor assembly 10 down the line, etc. The mounting of each pressure sensor 14 is still one-sided, even if the PCB continues underneath. Similarly, there also would only be a one-sided attachment of each sensor housing 1 to the printed circuit board 12. Alternatively, another flexible printed circuit board arranged underneath a first sensor assembly might be provided for a second sensor assembly.

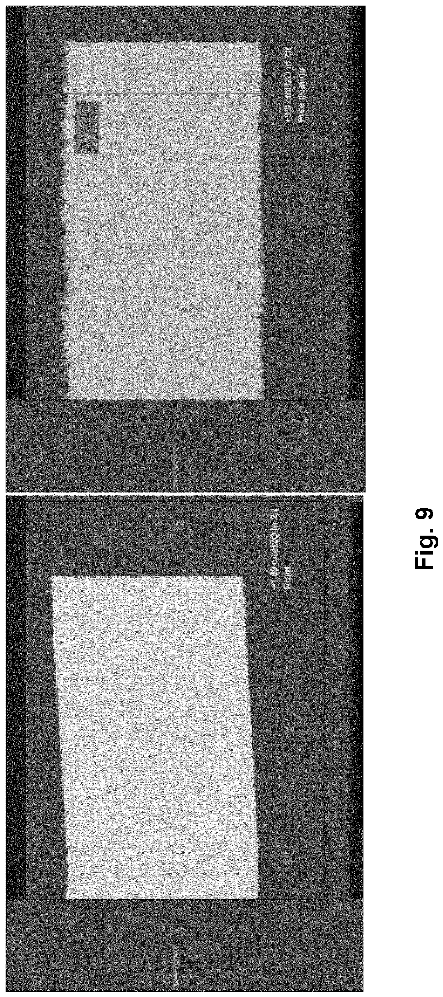

Figure 9 shows exemplary comparative pressure data showing sensor drift within the first two hours following exposure to a bench setup, exposed to pressure swings within an environment of 100% Relative Humidity (RH) and 37 degrees Celsius.

The left window shows 1,09 cmH2O drift with a prior art embodiment as schematically shown in Figure 2, whereas the right window shows 0,3 cmH2O drift with an embodiment as schematically shown Figure 5 and 6.

A pressure sensor assembly 1 of the prior art was measured with 10 samples, resulting in an average drift of 1,0 cmH2O with a standard deviation of 1,3 cmH2O, and limits of agreement ranging from -3,5 to 5,4 cmH2O, having an error range of 8,9 cmH2O.

A pressure sensor 10 assembly according to the present invention as shown in figure 5 and 6 was measured with 16 samples, resulting in an average drift of 1,0 cmH2O with a standard deviation of 0,7 cmH2O, and limits of agreement ranging from -1,0 to 3,0, having an error range of 4,1 cmH2O.

Accordingly, the change from a pressure sensor assembly according to the prior art to the invention resulted in a 54% reduction in error range, as shown in the table below:

| | Mean | SD | Lower Limit | Upper Limit | Error range | Improvement |
|---|---|---|---|---|---|---|
| Rigid (n=10) | 1,0 | 1,3 | -3,5 | 5,4 | 8,9 | 54% |
| Free floating (n=16) | 1,0 | 0,7 | -1,0 | 3,0 | 4,1 | |

First line: Pressure sensor assembly of the prior art
Second line: Pressure sensor assembly according to Figure 5/6.

## Claims

1. A pressure sensor assembly (10) for measuring the pressure of a fluid in a bodily duct of a patient, in particular for measurement of a gas pressure, the sensor assembly (10) comprising a sensor mount (12) and at least one pressure sensor (14) mounted on the sensor mount (12), **characterized in that** at least one end region (15) of the pressure sensor (14) is not supported by said sensor mount (12).

2. The pressure sensor assembly (10) according to claim 1,
wherein the sensor mount (12) is formed by a printed circuit board.

3. The pressure sensor assembly (10) according to claim 2,
wherein the printed circuit board is flexible and the pressure sensor (14) is directly mounted one-sided with a die attach adhesive (24), preferably having a Shore D hardness of 75 to 90.

4. The pressure sensor assembly (10) according to claim 2,
wherein the printed circuit board is rigid and the pressure sensor (14) is directly mounted one-sided with a die attach adhesive (24), preferably having a Shore D hardness of 50 to 80.

5. The pressure sensor assembly (10) according to one of the claims 2 to 4, wherein the pressure sensor (14) is connected to the printed circuit board with bond wires (11), the bond wires (11) being covered with a flexible glob top material (23), preferably a material having a Shore D hardness of below 80, more preferable below 65 and most preferable below 55.

6. The pressure sensor assembly (10) according to any of the previous claims, wherein the pressure sensor (14) has a proximal end (17) and a distal end (16) and wherein the pressure sensor (14) is mounted on the sensor mount (12) with its proximal end (17) and is arranged unsupported at its distal end (16).

7. The pressure sensor assembly (10) according to any of the previous claims, wherein the pressure sensor assembly (10) further comprises a sensor housing (19) at least partially encasing the pressure sensor (14).

8. The pressure sensor assembly according to 7, wherein the sensor housing (19) is provided with an opening (21) directed towards a measuring region (R) and wherein a measuring surface (18) of the pressure sensor (14) is arranged within said opening (21) so that the measuring surface (18) of the pressure sensor (14) is impinged by the pressure in the measuring region (R).

9. The pressure sensor assembly (10) according to claim 7 or 8, wherein the pressure sensor (14) is sealingly encased by the sensor housing (19), in particular via a flexible adhesive material (20), preferably a silicone, typically having a Shore A hardness of 15 - 20.

10. The pressure sensor assembly (10) according to claim 8 or 9, wherein a periphery (22) of the measuring surface (1) is sealingly mounted within the opening (21).

11. A pressure sensor assembly (10) for measuring the pressure of a fluid in a bodily duct of a patient, in particular for measurement of a gas pressure, the sensor assembly comprising a sensor mount (12), a pressure sensor (14) mounted on the sensor mount (12) and a sensor housing (19) at least partially encasing the pressure sensor (14), in particular a pressure sensor assembly (10) according to any of the claims 8 to 10,
wherein the sensor housing (19) is arranged in such a way that at least one end region of the housing (19) is not supported,
preferably wherein the sensor housing (19) is connected to the sensor mount (12) only at one end region (26), preferably with an adhesive (13).

12. The pressure sensor assembly (10) according to any of the previous claims, wherein the pressure sensor (14) is a MEMS device.

13. A catheter (30) for insertion into a bodily cavity of a patient and for measuring the pressure in a measuring region (R) of said bodily cavity, the catheter (30) having a wall (31) defining at least one inner lumen (32), wherein the catheter (30) comprises at least one pressure sensor assembly (10) according to one of the previous claims arranged such that it can be impinged by the pressure in the measuring region (R).

14. The catheter (30) according to claim 13, wherein the wall (31) of the catheter is provided with a least one window (33) and wherein the pressure sensor assembly (10) is arranged within the lumen (32) of the catheter (30) adjacent to the window (33) such that a measuring surface (18)of the pressure sensor (14) is impinged by the pressure in the measuring region (R).

15. The catheter (30) according to claim 13 or 14, wherein the catheter (30) is provided with electrical conductors (34) arranged within said lumen (32) for connecting the at least one pressure sensor assembly (10) to a measuring unit (40) arranged outside of the patient's body.

16. The catheter according to one of the claims 13 to 15,
wherein the catheter (30) is an endotracheal tube, an esophageal tube or a gastric tube or combinations thereof.
